# FASCICULE DE BREVET EUROPEEN

(11) **EP 2 134 675 B1**
(45) Date de publication et mention de la délivrance du brevet: **06.07.2011**
(21) Numéro de dépôt: 08788094.4
(22) Date de dépôt: 01.04.2008
(51) Int. Cl.: C07C 227/04, C07D 317/20

(54) **PROCEDE DE CO-PRODUCTION DE CARBONATES CYCLIQUES ET D'AMINO-ACIDES**
VERFAHREN ZUR GLEICHZEITIGEN HERSTELLUNG VON ZYKLISCHEN CARBONATEN UND AMINOSÄUREN
METHOD FOR THE CO-PRODUCTION OF CYCLIC CARBONATES AND AMINO ACIDS

(30) Priorité: 06.04.2007 FR 0754349
(43) Date de publication de la demande: 23.12.2009
(73) Titulaire: ARKEMA FRANCE, 92700 Colombes (FR)
(72) Inventeur: DUBOIS, Jean-Luc, 69390 Millery (FR)
(74) Mandataire: Sauvageot, Olivier Roger
(86) Numéro de dépôt international: PCT/FR2008/050568
(87) Numéro de publication internationale: WO 2008/139079

(56) Documents cités:
- EP-A- 0 955 298
- AELION: FIBRES, ENG. AND CHEM., vol. 17, no. 3, 1956, pages 79-82, XP009094483

## Description

L'invention vise un procédé conjugué de synthèse d'une part d'acides ω-amino-alcanoïques ou d'esters de ces acides et d'autre part de carbonates de polyols, à partir d'acides gras naturels.

L'évolution actuelle en matière d'environnement conduit dans les domaines de l'énergie et de la chimie à privilégier l'exploitation des matières premières naturelles provenant d'une source renouvelable. Ces contraintes environnementales imposent également d'éviter le transport et le stockage de matières premières dangereuses.

Un exemple de procédé industriel utilisant un acide gras comme matière première est celui de la fabrication à partir de l'acide ricinoléique extrait de l'huile de ricin, de l'acide amino-11-undécanoïque, qui est à la base de la synthèse du Rilsan^{®} 11. Ce procédé est décrit dans l'ouvrage « Les Procédés de Pétrochimie » de A. Chauvel et al. paru aux Editions TECHNIP (1986), et dans l'article R. Aelion Fibres (Engineering and Chemistry) (1956) 17, 79-82. L'acide amino-11-undécanoïque est obtenu en plusieurs étapes. La première consiste en une méthanolyse de l'huile de ricin en milieu basique produisant le ricinoléate de méthyle qui est ensuite soumis à une pyrolyse pour obtenir, d'une part l'heptanaldéhyde et, d'autre part l'undécylénate de méthyle. Ce dernier est passé en forme acide par hydrolyse. Ensuite l'acide formé est soumis à une hydrobromuration pour donner l'acide ω-bromé d'où l'on passe par amination à l'acide amino-11-undécanoïque.

On ne trouve pas dans la littérature d'autres documents décrivant la synthèse de composés du type ω-aminoacides à partir d'acides gras naturels. La demanderesse a récemment déposé une demande de brevet français sous le numéro FR 07.53285 décrivant des procédés de synthèse de ces ω-aminoacides à partir d'acides/esters gras naturels en mettant en oeuvre une réaction de métathèse croisée notamment avec l'acrylonitrile.

Le procédé de synthèse de l'acide amino-11-undécanoïque, opéré de manière industrielle depuis plusieurs décennies donne globalement satisfaction. Cependant, il présente un certain nombre d'inconvénients. Le premier inconvénient est que sa mise en oeuvre est en pratique asservie à l'accès à une matière première spécifique, l'huile de ricin. Le deuxième inconvénient est lié aux réactifs utilisés, l'ammoniac et le brome en particulier, qui nécessitent des précautions de stockage et d'utilisation coûteuses.

Le procédé produit lors de sa première étape du glycérol ainsi que lors des étapes ultérieures de nombreux sous-produits qu'il faut valoriser séparément : de l'heptanaldéhyde, de l'estérol (mélange des esters d'acide gras non craqués) mais aussi du méthanol lors de l'étape d'hydrolyse. Ce méthanol est alors purifié et concentré pour être recyclé pour effectuer l'étape de méthanolyse initiale.

Il existe plusieurs voies de synthèse du carbonate de glycérol. Certains procédés utilisent le phosgène, d'autres une oxydation du glycérol en présence de CO, JP 6 157509 et US 5,359,094, d'autres encore une transestérification avec un autre carbonate, et finalement d'autres procédés utilisent une transestérification du glycérol avec l'urée.

Depuis de nombreuses années, le carbonate de glycérol est produit par transestérification avec du carbonate d'éthylène en catalyse homogène US 2,915,529, FR 2 733 232, US 5,091,543. D'autres procédés de synthèse de carbonate utilisent des catalyseurs hétérogènes US 4,691,041.

La demande de brevet EP 955 298 décrit un procédé de transestérification du glycérol par de l'urée, utilisant un catalyseur portant des sites acides de Lewis associé à un ou des contre ions anioniques d'hétéroatomes. Les expérimentations ont montré que le glycérol apporté pouvait être sous la forme de glycérine de qualité technique.

Le brevet US 6,495,703 décrit aussi un procédé de production de carbonate de glycérol par transestérification de l'urée. La réaction est effectuée de préférence en présence d'un agent déshydratant.

Il faut noter que les réactions de carbonatation utilisant de l'urée ont déjà été décrites pour d'autres polyols EP 443 758 et EP 581 131.

Dans le procédé de synthèse d'acides ω-amino-alcanoïques ou d'esters de ces acides, il est nécessaire d'importer et de stocker des quantités importantes d'ammoniac, ce qui représente outre le problème du transport, un risque majeur en cas de fuite, non seulement pour le personnel mais aussi pour le voisinage. Ce stockage impose par conséquent des contraintes importantes au site exploitant ce type de procédé.

Le problème à résoudre est de limiter ces risques environnementaux tout en maintenant les performances du procédé ou même mieux en les améliorant.

L'invention vise donc un procédé conjugué de production d'acides ω-amino-alcanoïques et de carbonates de polyol à partir d'un acide gras naturel mono insaturé dans lequel le même acide gras naturel servira de matière première pour la synthèse d'acides ω-amino-alcanoïques d'une part, et de carbonates de polyol d'autre part. La synthèse des carbonates de polyol est réalisée par action de l'urée sur le polyol, l'ammoniac ainsi produit pourra être utilisé comme réactif pour l'étape d'amination lors de la dernière étape de la synthèse des acides ω-amino-alcanoïques.

L'invention vise un procédé conjugué de production d'acides ω-amino-alcanoïques et de carbonates de polyol à partir d'un acide gras naturel mono insaturé comportant les zones suivantes :
I) zone de méthanolyse d'une huile naturelle contenant un triglycéride d'au moins un acide gras mono insaturé produisant d'une part le méthanolate de l'acide gras répondant à la formule générale CH₃-(CH₂)ₙ-CHR-CH₂-CH=CH-(CH₂)ₚ-COOCH₃, dans laquelle R est soit H soit OH et n et p, identiques ou différents, sont des indices compris entre 2 et 11, et d'autre part du glycérol,
II) zone de synthèse d'un acide ω-amino-alcanoïque comportant les étapes suivantes a) transformation tout d'abord du méthanolate de l'acide gras en un ester gras ω-insaturé, transformation réalisée soit par éthénolyse du méthanolate pour obtenir un composé de formule CH₂=CH-(CH₂)ₚ-COOCH₃, soit par pyrolyse d'un méthanolate répondant à la formule définie en I) avec R=OH et pour obtenir un composé de formule CH₂=CH-(CH₂)ₚ₊₁-COOCH₃, suivie b) d'une hydrolyse de l'ester issu de l'étape a), puis c) d'une hydrobromuration de l'acide ω-insaturé issu de l'étape b), puis d) d'une amination à l'ammoniac réalisée généralement avec de l'ammoniaque (solution aqueuse d'ammoniac) du composé résultant de l'hydrobromuration de l'étape c),
III) zone de synthèse d'un carbonate de polyol par réaction de l'urée soit directement sur le glycérol, soit sur du propylène glycol ou de l'éthylène glycol obtenus suite à une réduction par hydrogénation du glycérol, la réaction du polyol avec l'urée produisant de l'ammoniac,
IV) zone de récupération de l'ammoniac issue de la zone III) ainsi que celle issue de la zone IId où la réaction est réalisée avec un excès d'ammoniac, pour servir d'alimentation pour l'amination de l'étape d) de la zone II.

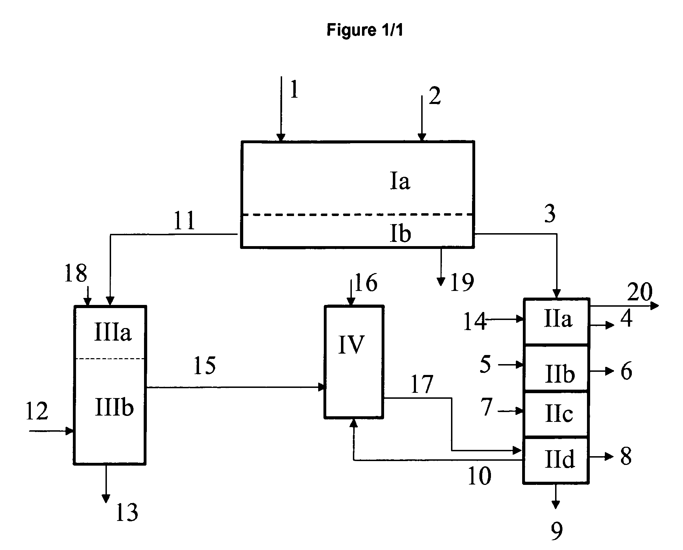

Le procédé de l'invention est décrit en référence au schéma simplifié annexé en figure 1.

La zone I est alimentée par la ligne 1 en huile naturelle contenant un triglycéride d'au moins un acide gras mono insaturé d'une part et d'autre part en méthanol par la ligne 2. Une fois la réaction d'estérification de l'acide gras réalisée dans la zone la selon la réaction suivante : 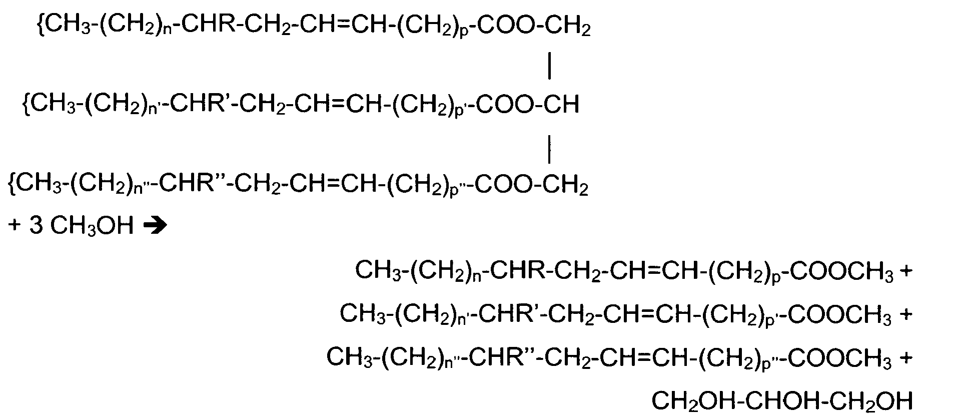 R, R', R" étant soit H soit OH et n, n', n", identiques ou différents et p, p', p", identiques ou différents, des indices compris entre 2 et 11,
on sépare en général par décantation dans la zone Ib d'une part l'ester méthylique de l'acide qui est alors adressé par la ligne 3 à la zone II et d'autre part le glycérol qui est adressé par la ligne 11 à la zone III. Les composés résiduels sont extraits de la zone I par la ligne 19.
L'ester méthylique est alors introduit par la ligne 3 dans la zone IIa où il est transformé en un ester gras ω-insaturé de longueur de chaîne réduite par rapport à celle de l'acide gras de la charge initiale. Cette transformation est obtenue soit par pyrolyse, soit par éthénolyse.
Dans le premier cas, la chaleur nécessaire à la réaction de pyrolyse qui correspond au processus réactionnel suivant

CH₃-(CH₂)ₙ-CHOH-CH₂-CH=CH-(CH₂)ₚ-COOCH₃ + Δ → CH₂=CH-(CH₂)ₚ₊₁-COOCH₃+ CH₃-(CH₂)ₙ-CHO

est introduite par la ligne 14, sous forme de vapeur d'eau surchauffée, puis extraite par la ligne 20. L'aldéhyde co-produit de la réaction, par exemple l'heptanal dans le cas du ricinoléate de méthyle, est extrait par la ligne 4.
Dans le second cas, on introduit dans la zone IIa par la ligne 14, de l'éthylène qui réagit avec l'ester insaturé, selon le processus illustré ci-après, CH₃-(CH₂)ₙ-CH=CH-(CH₂)ₚ-COOCH₃ + CH₂=CH₂ ⇔

CH₂=CH-(CH₂)ₚ-COOCH₃ + CH₂=CH-(CH₂)ₙ-CH₃

pour former l'ester gras ω-insaturé d'une part et une α-oléfine d'autre part. L'α-oléfine formée est extraite par la ligne 4.
L'ester gras ω-insaturé formé en zone IIa est ensuite transféré en zone IIb où il est soumis à une hydrolyse afin d'obtenir l'acide gras ω-insaturé correspondant. L'eau est introduite par la ligne 5 et le méthanol extrait par la ligne 6. Ce méthanol, éventuellement purifié, pourra être recyclé en zone I pour l'estérification de l'acide gras de la charge.
L'acide gras ω-insaturé issu de la zone IIb est introduit dans la zone IIc où il est soumis à une hydrobromuration pour obtenir l'acide ω-bromo-alcanoïque selon le processus réactionnel suivant :

CH₂=CH-(CH₂)ₚ-COOH + HBr → Br CH₂-(CH₂)ₚ₊₁-COOH

L'acide bromhydrique est introduit par la ligne 7.
L'acide ω-bromo-alcanoïque issu de la zone IIc est introduit dans la zone IId et est soumis à une amination par l'ammoniac selon le processus réactionnel suivant :

Br CH₂-(CH₂)ₚ₊₁-COOH + NH₃ → NH₂-(CH₂)ₚ₊₂-COOH + HBr

L'ammoniac est introduit dans la zone IId par la ligne 17. L'acide ω-amino alcanoïque est extrait de la zone II par la ligne 9. L'acide bromhydrique formé, en pratique le bromure d'ammonium (NH₄Br) dans la mesure où cette opération est réalisée avec un excès d'ammoniac est extrait par la ligne 8 et l'excès d'ammoniac renvoyé à la zone IV par la ligne 10. En général, l'ammoniac est pour toutes ces opérations sous forme de solution aqueuse.
Le glycérol issu de la zone I est adressé par la ligne 11 à la zone III dans laquelle on injecte par la ligne 12 de l'urée pour former le carbonate de glycérol selon le processus réactionnel suivant : 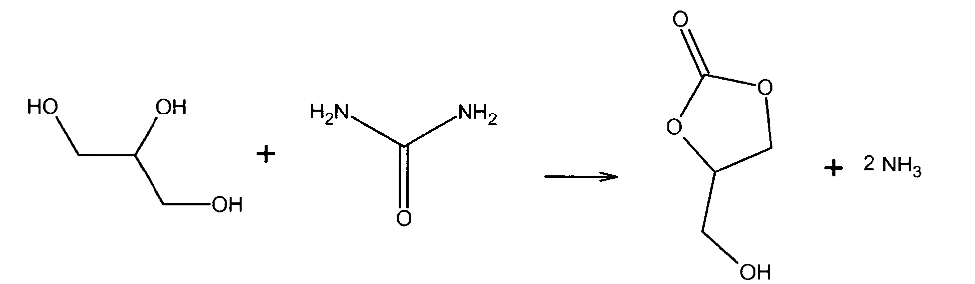 Le carbonate de glycérol synthétisé est extrait de la zone III par la ligne 13 et l'ammoniac formé est adressée à la zone IV par la ligne 15.

Dans le cas où l'on souhaite synthétiser un carbonate cyclique de propylène glycol 1,2 ou 1,3, la zone III est divisée en deux zones IIIa et IIIb; dans la zone IIIa on procède à une réduction partielle du glycérol par injection d'hydrogène par la ligne 18 et le (ou les) propylène glycol(s) sont transférés, après avoir été séparé(s) de l'eau produite par la réaction d'hydrogénation, dans la zone IIIb où a lieu la réaction avec l'urée pour former les carbonates de propylèneglycol cycliques à 5 ou 6 atomes selon la structure du propylène glycol ayant réagi.

Si l'on souhaite obtenir des carbonates d'éthylène glycol, il est possible de procéder en modifiant les conditions opérationnelles au niveau de la zone IIIa à une hydrogénolyse du glycérol dans des conditions plus sévères produisant de l'éthylène glycol qui est transformé par action de l'urée en un carbonate d'éthylène, cycle à 5 atomes. En pratique, les opérations d'hydrogénation du glycérol donnent le plus souvent un mélange de propylène glycol et d'éthylène glycol.

La zone IV comporte outre les lignes d'alimentation 15 et 10, une ligne d'appoint 16 permettant notamment de fournir l'ammoniac nécessaire au démarrage de l'unité et à sa régulation en cas de variation de la production d'ammoniac au niveau de la zone III.

Le procédé est particulièrement bien adapté à la synthèse de l'acide amino-11-undécanoïque et de carbonates de glycérol à partir de l'huile de ricin.
Les différentes réactions seront les suivantes :

Zone I: Huile de ricin + CH₃OH → 3 CH₃-(CH₂)₅-CHOH-CH₂-CH=CH-(CH₂)₇-COOCH₃ + CH₂OH-CHOH-CH₂OH

Zone IIa : CH₃-(CH₂)₅-CHOH-CH₂-CH=CH-(CH₂)₇-COOCH₃ + Δ → CH₂=CH-(CH₂)₈-COOCH₃ + CH₃-(CH₂)₅-CHO

Zone IIb : CH₂=CH-(CH₂)₈-COOCH₃ + H₂O → CH₂=CH-(CH₂)₈-COOH + CH₃OH

Zone IIc : CH₂=CH-(CH₂)₈-COOH + HBr → BrCH₂-(CH₂)₉-COOH

Zone IId : BrCH₂-(CH₂)₉-COOH + 2 NH3 → NH₂- (CH₂)₁₀COOH + NH₄Br

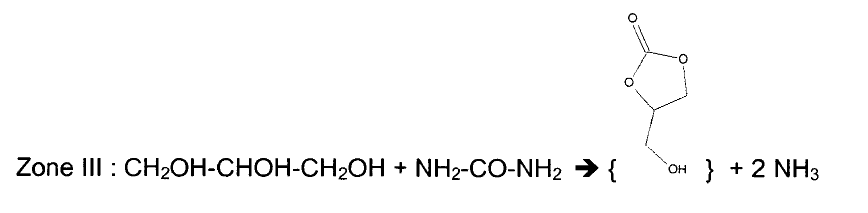

On pourra utiliser exactement le même procédé avec une réaction de pyrolyse dans la zone IIa pour la synthèse de l'acide amino-13-tridécanoïque en utilisant pour charge une huile (ou triglycéride) contenant l'acide lesquérolique répondant à la formule suivante :

CH₃-(CH₂)₅-CHOH-CH₂-CH=CH-(CH₂)₉-COOH

Pour la synthèse d'autres ω-aminoacides, on aura le plus souvent recours lors de l'étape IIa à une réaction de métathèse avec l'éthylène (ou éthénolyse).

Pour illustrer le mécanisme réactionnel en prenant pour exemple la synthèse de l'acide amino-7-heptanoïque, on utilisera comme matière première d'acide gras naturel, l'acide pétrosélinique (acide cis-6-octadécènoïque). On le soumet à une éthénolyse sous sa forme ester ou acide (R=H) selon le processus suivant.

CH₃-(CH₂)₁₀-CH=CH-(CH₂)₄-COOR + CH₂=CH₂ ⇔ CH₂=CH-(CH₂)₄-COOR + CH₂=CH-(CH₂)₁₀-CH₃

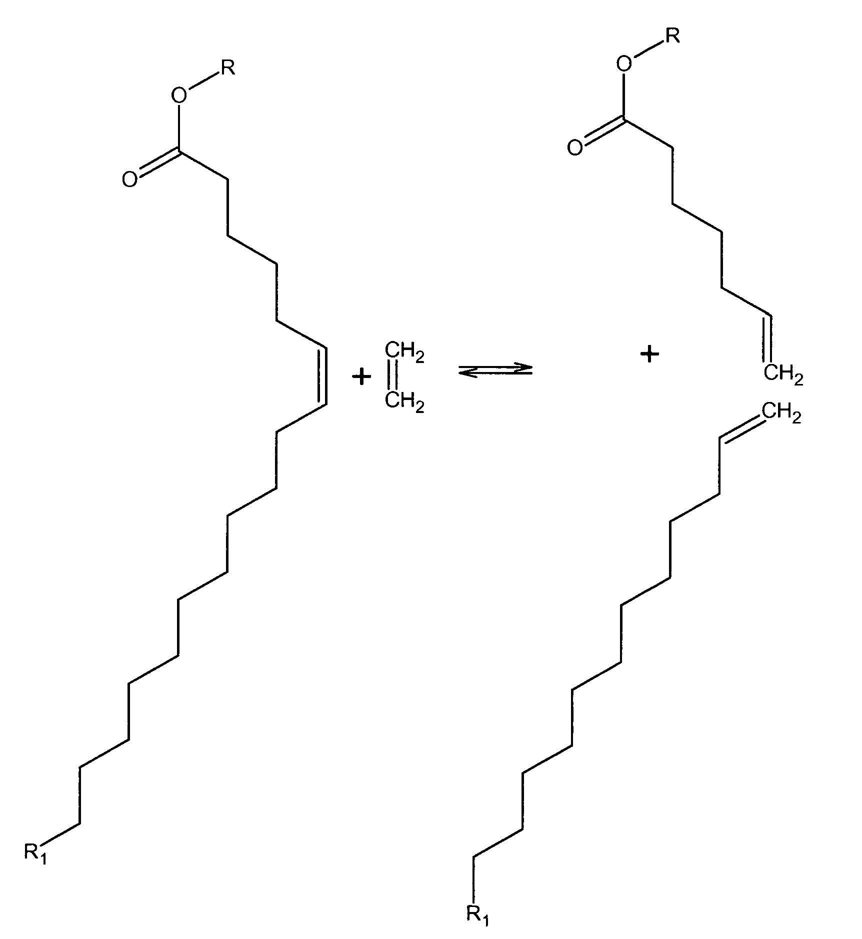

On peut observer à ce sujet que l'ordre des étapes IIa et IIb de la zone II peut être alors inversé.

En utilisant le même processus, on pourra synthétiser divers ω-amino acides. On peut citer l'acide amino-6-hexanoïque en utilisant une charge contenant de l'acide lauroléique (acide cis-5-lauroléique). De même on pourra synthétiser l'acide amino-10-décanoïque en utilisant une charge contenant des acides tels que les acides oléique (acide cis-9-octadécènoïque), palmitoléique (acide cis-9-hexadécènoïque), gadoléique (acide cis-9-eicosènoïque) et myristoléique (acide cis-9-tetradécènoïque). Pour la synthèse de l'acide amino-12-dodécanoïque, les charges utilisables sont celles contenant les acides vaccénique (acide cis-11-octadécènoïque), gondoïque (acide cis-11-eicosènoïque) et cétoléique (acide cis-11-docosènoïque). Enfin pour la synthèse de l'acide amino-14-tétradécanoïque, on pourra utiliser une charge contenant l'acide érucique (acide cis-13-docosènoïque).

Les conditions de mise en oeuvre des différentes étapes des zones I, II, III et IV sont connues de l'homme de l'art.
On peut cependant préciser que :
- la méthanolyse de la zone I est généralement réalisée par exemple à une température comprise entre 60 et 100 °C en présence d'un catalyseur du type méthylate de sodium et avec un excès de méthanol. Il est aussi possible d'utiliser une catalyse hétérogène à plus haute température - ou même une catalyse acide.
- la pyrolyse de l'étape a) de la zone II est conduite à une température généralement comprise entre 400 et 600 °C.
- pour la métathèse de l'étape a) de la zone II, tout catalyseur de métathèse actif et sélectif pourra être utilisé. On utilisera cependant de préférence des catalyseurs à base de ruthénium.
- la réaction d'hydrolyse de l'étape b) de la zone II est réalisée par exemple à la température ambiante ou même à une température un peu plus élevée.
- l'hydrobromuration de l'étape c) de la zone II est réalisée à basse température en présence d'un solvant organique (par exemple le toluène).
- l'amination de l'étape d) de la zone II est réalisée à température ambiante en présence d'ammoniac en excès dans une solution aqueuse.
- la conversion du glycérol en carbonate de glycérol réalisée au niveau de la zone III est comme cela a été indiqué précédemment connue depuis plusieurs d'années. Pour la conduite du procédé, la solution retenue est la transestérification du glycérol par l'urée. Cette réaction est conduite en présence de catalyseurs connus en soi par exemple ceux décrits dans les brevets USP 6,495,703 oxyde de zinc, ou EP 0 955 298., sulfates métalliques, de zinc ou organométalliques.

Dans une variante, le procédé permet d'obtenir des carbonates de polyols autres que le glycérol à savoir les diols comportant 3 atomes de carbone tels le propanediol-1,2 (ou propylèneglycol-1,2), le propanediol-1,3 et l'éthylène glycol (ou éthanediol) et notamment le propylèneglycol-1,2 qui sont obtenus par hydrogénation du glycérol issu de la zone I. Cette hydrogénation est conduite à des conditions bien connues de l'homme de l'art.

Plusieurs demandes de brevets et articles décrivent les conditions pour effectuer la transformation par hydrogénation du glycérol en propylène glycol, propanediol et éthylène glycol. On peut citer les brevets US 5,276,181, US 5,214,219, US 5,616,817, US 4,642,394 et les articles suivants : Daniel G. Lahr et col. J.Catal. 232 (2005), 386-394, Daniel G. Lahr et col. Ind. Eng. Chem. Res. (2003) 42,(22),5467-5472. D'autre part, des voies utilisant aussi des procédés de fermentation ont été décrites pour la transformation du glycérol en propane diol et propylène glycol, par exemple dans les article suivants : D.C. Cameron et col. Biotechnology 4 (1986), 651, D.C. Cameron et col. Biotechnol. Prog. 14 (1998), 116.
Le brevet US 4,642,394 décrit un procédé de production d'un mélange de propanediols 1,2 et 1,3, à partir de glycérol par action d'un gaz de synthèse (H₂ + CO) dans un milieu solvant organique en présence d'un catalyseur soluble à base de tungstène et d'un métal du Groupe VIII.
Le brevet US 5,214,219 décrit un procédé de production d'un mélange de 1,2 propanediol et d'éthanediol à partir de glycérol par hydrogénation du glycérol en présence d'un catalyseur hétérogène zinc-cuivre dans un milieu aqueux ou solvant alcoolique.
Le brevet US 5,616,817 décrit un procédé de production de 1,2 propanediol partir de glycérol en présence d'un catalyseur hétérogène à base de cobalt, de cuivre, de manganèse et de molybdène.
La transestérification du propanediol-1,2 par l'urée pour la synthèse du carbonate de propylène est décrite par Xinqiang Zhao et al dans un article intitulé « Synthesis of Propylene Carbonate from Urea and 1,2-Propylene Glycol over a Zinc Acetate Catalyst » Ind. Eng. Chem. Res., 43 (15), 40384042,2004. Selon les mêmes auteurs dans un article paru dans Journal of Chemical Technology & Biotechnology en 2006 la même réaction peut être conduite avec un catalyseur oxyde double zinc-fer.
La transestérification de l'éthanediol avec l'urée a été décrite par Chiyoda et Mitsubishi Gas Chemical dans une publication de Chiyoda sur la synthèse de carbonate d'éthylène. (ACS 212th National Meeting, August 1996, Div Fuel Chem. Preprint, Vol 41, N°3, pp868-872). Des données expérimentales citées dans un brevet de Mistubishi Gas Chemical (US 5,489,702) montrent qu'à 145°C et sous 100 mm Hg, avec 2 h de temps de contact, et en utilisant un catalyseur oxyde de zinc, une conversion complète de l'urée est obtenue pour une sélectivité en carbonate d'éthylène supérieure à 97 % par rapport à l'urée comme par rapport à l'éthylène glycol.

## Revendications

1. Procédé conjugué de production d'acides ω-amino-alcanoïques et de carbonates de polyol à partir d'un acide gras naturel mono insaturé comportant les zones réactionnelles suivantes :
I) zone de méthanolyse d'une huile naturelle contenant un triglycéride d'au moins un acide gras mono insaturé introduite par la ligne 1 par le méthanol introduit par la ligne 2 produisant au sein de la zone la, d'une part le méthanolate de l'acide gras répondant à la formule générale CH₃-(CH₂)ₙ-CHR-CH₂-CH=CH-(CH₂)ₚ-COOCH₃, dans laquelle R est soit H soit OH et n et p, identiques ou différents, sont des indices compris entre 2 et 11, et d'autre part du glycérol qui sont respectivement adressés, après séparation au sein de la zone Ib aux zones II par la ligne 3 et zone III par la ligne 11,
II) zone de synthèse d'un acide ω-amino-alcanoïque comportant les étapes suivantes a) transformation tout d'abord du méthanolate de l'acide gras, introduit par la ligne 3 en un ester gras ω-insaturé, transformation réalisée, soit par éthénolyse du méthanolate, l'éthylène étant introduit par la ligne 14 pour obtenir un composé de formule CH₂=CH-(CH₂)ₚ-COOCH₃, l'a-oléfine co-produite étant extraite par la ligne 4, soit par pyrolyse au moyen de vapeur d'eau surchauffée introduite par la ligne 14, d'un méthanolate répondant à la formule définie en I) avec R=OH et pour obtenir un composé de formule CH₂=CH-(CH₂)ₚ₊₁-COOCH₃, l'aldéhyde co-produit de la réaction étant extrait par la ligne 4, suivie b) d'une hydrolyse de l'ester issu de l'étape a) par introduction d'eau par la ligne 5, le méthanol alors formé étant extrait par la ligne 6, puis c) d'une hydrobromuration de l'acide ω-insaturé issu de l'étape b) par action d'acide bromhydrique introduit par la ligne 7, puis d) d'une amination à l'ammoniac, introduite par la ligne 17, du composé résultant de l'hydrobromuration de l'étape c) pour former l'acide ω-amino-alcanoïque extrait par la ligne 9 avec élimination de l'acide bromhydrique, sous forme de sel d'ammonium par la ligne 8 et le recyclage de l'ammoniac excédentaire à la zone IV par la ligne 10,
III) zone de synthèse d'un carbonate de polyol par réaction de l'urée soit directement sur le glycérol amené par la ligne 11, soit sur du propanediol-1,3, du propylène glycol ou de l'éthylène glycol, obtenus suite à une réduction par hydrogénation du glycérol, la réaction du polyol avec l'urée produisant de l'ammoniac, le carbonate de polyol étant extrait par la ligne 13 et l'ammoniac formé adressée à la zone IV par la ligne 15,
IV) zone de récupération de l'ammoniac issue de la zone III par la ligne 15 ainsi que celle par la ligne 10 issue de la zone IId où la réaction est réalisée avec un excès d'ammoniac, pour servir d'alimentation par la ligne 17 pour l'amination de l'étape d) de la zone II.

2. Procédé selon la revendication 1 **caractérisé en ce que** la réaction de l'étape de la zone IIa est une pyrolyse de l'ester de l'acide de formule CH₃-(CH₂)ₙ- CHOH-CH₂-CH=CH-(CH₂)ₚ-COOCH₃, dans laquelle n et p, identiques ou différents, sont des indices compris entre 2 et 11 pour obtenir un composé de formule CH₂=CH-(CH₂)ₚ₊₁-COOCH₃

3. Procédé selon la revendication 1 **caractérisé en ce que** la réaction de l'étape de la zone IIa est une éthénolyse du méthanolate de l'acide gras répondant à la formule générale CH₃-(CH₂)ₙ-CHR-CH₂-CH=CH-(CH₂)ₚ-COOCH₃ pour obtenir un composé de formule CH₂=CH-(CH₂)ₚCOOCH₃.

4. Procédé selon l'une des revendications 1 à 3 **caractérisé en ce que** le glycérol issu de la zone I est adressé à la zone III est transformé en carbonate de glycérol par action de l'urée.

5. Procédé selon l'une des revendications 1 à 3 **caractérisé en ce que** le glycérol issu de la zone I est adressé à la zone III est réduit dans une zone IIIa par action de l'hydrogène en au moins un diol, propanediol et/ou éthanediol, ces derniers étant ensuite transformés en carbonates de propanediol et/ou éthanediol par action de l'urée.

## Claims

1. Combined process for the production of ω-aminoalkanoic acids and polyol carbonates from a natural monounsaturated fatty acid, comprising the following reaction regions:
I) region of methanolysis of a natural oil comprising a triglyceride of at least one monounsaturated fatty acid, introduced via line 1, by methanol, introduced via line 2, producing, within the region Ia, on the one hand, the methyl ester of the fatty acid corresponding to the general formula CH₃-(CH₂)ₙ-CHR-CH₂=CH-(CH₂)ₚ-COOCH₃, in which R is either H or OH and n and p, which are identical or different, are indices between 2 and 11, and, on the other hand, glycerol, which are respectively sent, after separation within the region Ib, to the region II, via the line 3, and the region III, via the line 11,
II) region of synthesis of an ω-aminoalkanoic acid, comprising the following stages: a) first of all conversion of the methyl ester of the fatty acid, introduced via the line 3, to an ω-unsaturated fatty ester, which conversion is carried out either by ethenolysis of the methyl ester, the ethylene being introduced via the line 14, in order to obtain a compound of formula CH₂=CH-(CH₂)ₚ-COOCH₃, the α-olefin coproduced being extracted via the line 4, or by pyrolysis, by means of superheated steam introduced via the line 14, of a methyl ester corresponding to the formula defined in I) with R=OH, in order to obtain a compound of formula CH₂=CH-(CH₂)ₚ₊₁-COOCH₃, the aldehyde coproduced in the reaction being extracted via the line 4, followed b) by a hydrolysis of the ester resulting from stage a) by introduction of water via the line 5, the methanol then formed being extracted via the line 6, then c) by a hydrobromination of the ω-unsaturated acid resulting from stage b) by the action of hydrobromic acid, introduced via the line 7, then d) by an amination with ammonia, introduced via the line 17, of the compound resulting from the hydrobromination of stage c), in order to form the ω-aminoalkanoic acid, extracted via the line 9, with removal of the hydrobromic acid, in the ammonium salt form, via the line 8 and the recycling of the excess ammonia to the region IV via the line 10,
III) region of synthesis of a polyol carbonate by reaction of urea, either directly with the glycerol, introduced via the line 11, or with 1,3-propanediol, propylene glycol or ethylene glycol, obtained subsequent to a reduction by hydrogenation of the glycerol, the reaction of the polyol with the urea producing ammonia, the polyol carbonate being extracted with the line 13 and the ammonia formed being sent to the region IV via the line 15,
IV) region of recovery of the ammonia resulting from the region III, via the line 15, and that, via the line 10, resulting from the region IId where the reaction is carried out with an excess of ammonia, in order to act as feed, via the line 17, for the amination of stage d) of the region II.

2. Process according to Claim 1, **characterized in that** the reaction of the stage of the region IIa is a pyrolysis of the ester of the acid of formula CH₃-(CH₂)ₙ-CHOH-CH₂-CH=CH-(CH₂)ₚ-COOCH₃, in which n and p, which are identical or different, are indices of between 2 and 11, in order to obtain a compound of formula CH₂=CH-(CH₂)ₚ₊₁-COOCH₃.

3. Process according to Claim 1, **characterized in that** the reaction of the stage of the region IIa is an ethenolysis of the methyl ester of the fatty acid corresponding to the general formula CH₃-(CH₂)ₙ-CHR-CH₂-CH=CH-(CH₂)ₚ-COOCH₃, in order to obtain a compound of formula CH₂=CH-(CH₂)ₚCOOCH₃.

4. Process according to one of Claims 1 to 3, **characterized in that** the glycerol resulting from the region I and sent to the region III is converted to glycerol carbonate by the action of urea.

5. Process according to one of Claims 1 to 3, **characterized in that** the glycerol resulting from the region I and sent to the region III is reduced, in a region IIIa, by the action of hydrogen, to give at least one diol, propanediol and/or ethanediol, the latter products subsequently being converted to propanediol and/or ethanediol carbonates by the action of urea.

## Patentansprüche

1. Gemeinsames Verfahren zur Herstellung von ω-Aminoalkansäuren und Polyolcarbonaten, ausgehend von einer natürlichen, ungesättigten Monofettsäure, das die folgenden Reaktionszonen umfasst:
I) Zone der Methanolyse eines natürlichen Öls, das ein Triglycerid mindestens einer ungesättigten Monofettsäure enthält und durch Leitung 1 eingeleitet wird, mittels durch die Leitung 2 eingeleitetes Methanol, wobei in der Zone Ia einerseits das Methanolat der Fettsäure, das der allgemeinen Formel CH₃-(CH₂)ₙ-CHR-CH₂-CH=CH-(CH₂)ₚ-COOCH₃ entspricht, in der R entweder H oder OH ist und n und p, identisch oder verschieden, Indizes zwischen 2 und 11 sind, und andererseits Glycerin produziert wird, die nach der Trennung innerhalb der Zone Ib durch Leitung 3 in die Zone II bzw. durch die Leitung 11 in die Zone III schickt werden,
II) Zone der Synthese einer ω-Aminoalkansäure, welche die folgenden Schritte umfasst: a) zunächst die Umwandlung des durch Leitung 3 eingeleiteten Methanolats der Fettsäure in einen ω-ungesättigten Fettester, wobei die Umwandlung entweder durch Ethenolyse des Methanolats, wobei Ethylen über Leitung 14 eingeführt wird, um eine Zusammensetzung der Formel CH₂=CH-(CH₂)ₚ-COOCH₃ zu erhalten, wobei das Nebenprodukt α-Olefin über Leitung 4 abgezogen wird, oder durch Pyrolyse eines Methanolats, das der in I definierten Formel mit R=OH entspricht, mittels überhitztem, über Leitung 14 eingeleitetem Wasserdampf erfolgt, um eine Zusammensetzung der Formel CH₂=CH-(CH₂)ₚ₊₁-COOCH₃ zu erhalten, wobei das Nebenprodukt Aldehyd dieser Reaktion über Leitung 4 abgezogen wird; gefolgt von b) einer Hydrolyse des aus Schritt a) stammenden Esters durch Einleitung von Wasser über Leitung 5, wobei das gebildete Methanol über Leitung 6 abgezogen wird; anschließend c) eine Hydrobromierung der ω-ungesättigten Säure aus Schritt b) durch Wirkung von durch Leitung 7 eingeleiteter Bromwasserstoffsäure; schließlich d) eine Aminierung mit über Leitung 17 eingeleitetem Ammoniak der aus der Hydrobromierung in Schritt c) resultierenden Verbindung zur Bildung einer ω-Aminoalkansäure, die über Leitung 9 abgezogen wird, mit Eliminierung von Bromwasserstoffsäure in Form des Ammoniumsalzes über Leitung 8 und Rückführung des überschüssigen Ammoniaks über Leitung 10 zur Zone IV,
III) Zone der Synthese eines Polyolcarbonats durch Reaktion von Harnstoff entweder direkt mit dem über Leitung 11 zugeführten Glycerin oder mit 1,3-Propandiol, Propylenglykol oder Ethylenglykol, die als Folge einer Reduktion von Glycerin durch Hydrieren erhalten wurden, wobei die Reaktion von Polyol mit Harnstoff Ammoniak erzeugt, wobei das Polyolcarbonat über Leitung 13 abgezogen wird und das gebildete Ammoniak über Leitung 15 in Zone IV geschickt wird,
IV) Rückgewinnungszone für Ammoniak, das aus Zone III über Leitung 15 sowie über Leitung 10 aus der Zone IId stammt, wo die Reaktion mit einem Überschuss an Ammoniak durchgeführt wurde, um damit Leitung 17 für die Aminierung in Schritt d) der Zone II zu versorgen.

2. Verfahren nach Anspruch 1, **dadurch gekennzeichnet, dass** der Reaktionsschritt in der Zone IIa eine Pyrolyse des Säureesters der Formel CH₃-(CH₂)ₙ-CHOH-CH₂-CH=CH-(CH₂)ₚ-COOCH₃ ist, in der n und p, identisch oder verschieden, Indizes zwischen 2 und 11 sind, um eine Verbindung der Formel CH₂=CH-(CH₂)ₚ₊₁-COOCH₃ zu erhalten.

3. Verfahren nach Anspruch 1, **dadurch gekennzeichnet, dass** der Reaktionsschritt in der Zone IIa eine Ethenolyse des Methanolats der Fettsäure ist, das der allgemeinen Formel CH₃-(CH₂)ₙ-CHR-CH₂-CH=CH-(CH₂)ₚ-COOCH₃ entspricht, um eine Verbindung der Formel CH₂=CH-(CH₂)ₚCOOCH₃ zu erhalten.

4. Verfahren nach einem der Ansprüche 1 bis 3, **dadurch gekennzeichnet, dass** das aus Zone I stammende und in Zone III geschickte Glycerin durch Wirkung von Harnstoff in ein Glycerincarbonat umgewandelt wird.

5. Verfahren nach einem der Ansprüche 1 bis 3, **dadurch gekennzeichnet, dass** das aus Zone I stammende und in Zone III geschickte Glycerin in einer Zone IIIa durch die Wirkung von Wasserstoff zu mindestens einem Diol, Propandiol und/oder Ethandiol reduziert wird, wobei letztere anschließend durch Wirkung von Harnstoff in Propandiol- und/oder Ethandiolcarbonat umgewandelt werden.
